# EUROPEAN PATENT SPECIFICATION

(11) **EP 2 997 954 B1**
(45) Date of publication and mention of the grant of the patent: **22.01.2020**
(21) Application number: 14798119.5
(22) Date of filing: 24.04.2014
(51) Int. Cl.: A61K 8/02, A61K 8/27, A61K 8/29, A61K 8/88, A61Q 17/04, C08K 3/22

(54) **RESIN POWDER INCLUDING ULTRAVIOLET SCATTERING AGENT, PRODUCTION METHOD THEREFOR, AND COSMETIC**
HARZPULVER MIT EINEM ULTRAVIOLETTSTREUUNGSMITTEL, HERSTELLUNGSVERFAHREN DAFÜR UND KOSMETIKUM
POUDRE DE RESINE COMPRENANT UN AGENT DE DIFFUSION DES RAYONS ULTRAVIOLETS, PROCEDE DE PRODUCTION CORRESPONDANT, ET PRODUIT COSMETIQUE

(30) Priority: 14.05.2013 JP 2013102534
(43) Date of publication of application: 23.03.2016
(73) Proprietor: Daicel-Evonik Ltd., Tokyo 163-0913 (JP)
(72) Inventor: MUTSUDA Mitsuteru, Himeji-shi Hyogo 671-1281 (JP); IGUCHI Hirofumi, Himeji-shi Hyogo 671-1281 (JP)
(74) Representative: Grünecker Patent- und Rechtsanwälte PartG mbB
(86) International application number: PCT/JP2014/061596
(87) International publication number: WO 2014/185249

(56) References cited:
- EP-A1- 1 632 212
- EP-A1- 2 163 569
- JP-A- S 632 919
- JP-A- H08 269 433
- JP-A- 2003 238 340
- JP-A- 2005 179 249
- JP-A- 2008 081 455
- US-A1- 2011 268 674

## Description

### TECHNICAL FIELD

The present invention relates to resin powders including ultraviolet scattering agents (or ultraviolet protective agents), methods for producing the resin powders, and cosmetics containing the resin powders as defined in the claims.

### BACKGROUND ART

In order to protect the skin from ultraviolet rays, various cosmetics (e.g., sunscreens and UV foundation) have so far been developed. The most common technique is mixing of an ultraviolet scattering agent (such as titanium oxide or zinc oxide) as an additive in a cosmetic preparation.

The ultraviolet scattering agent has a higher ultraviolet-scattering effect as the agent has a smaller particle diameter. An ultraviolet scattering agent to be used for a cosmetic generally has a nanometer-order particle diameter. However, the ultraviolet scattering agent having a nanometer-order particle diameter has not been inspected completely for the harmful effects arising from the contact with the skin. In a different field, lung cancer due to asbestos in the form of nanowhiskers, has developed into a large social problem. With this as a start, the current tendency is that attention to the effects of such fine particles on the human body cannot be overpaid. For zinc oxide, a large quantity of zinc oxide contained in a cosmetic produces zinc ions that pseudo-crosslink additives in the cosmetic, thereby disadvantageously increasing the viscosity of the cosmetic.

JP H08 269433 discloses plate-like composites, wherein the matrix component is a perfluoropolyether.

Japanese Patent Application Laid-Open Publication No. 2013-56860 (JP-2013-56860A, Patent Document 1) discloses a skin cosmetic containing zinc oxide and titanium oxide as ultraviolet protective components, wherein the zinc oxide is enclosed in a matrix component to form a composite particle, and the matrix component is selected from a thermoplastic resin, a thermoplastic elastomer, and a rubber.

For the skin cosmetic disclosed in the document, since the zinc oxide is enclosed in the matrix component, direct contact of the zinc oxide with the skin can be prevented. Thus even if the zinc oxide is in the form of a nanoparticle, the skin cosmetic has an improved advantage in respect of the safety for the human body. Unfortunately, the zinc oxide enclosed in the matrix component tends to decrease in ultraviolet scattering function. In particular, the composite particle disclosed in the document tends to notably decreases in ultraviolet scattering function probably because the composite particle is in a spherical form and is easy to aggregate.

### RELATED ART DOCUMENTS

### PATENT DOCUMENTS

Patent Document 1: JP-2013-56860A (Claims and Examples)

### SUMMARY OF THE INVENTION

### PROBLEMS TO BE SOLVED BY THE INVENTION

It is therefore an object of the present invention to provide a resin powder having an ultraviolet scattering function, a method for producing the resin powder, and a cosmetic containing the resin powder.

Another object of the present invention is to provide a resin powder efficiently showing an ultraviolet scattering function even in a case where the resin powder comprises an ultraviolet scattering agent contained (or enclosed) in a resin, a method for producing the resin powder, and a cosmetic containing the resin powder.

It is still another object of the present invention to provide a resin powder having both high safety for the human body and excellent ultraviolet scattering function, a method for producing the resin powder, and a cosmetic containing the resin powder.

### MEANS TO SOLVE THE PROBLEMS

The inventors of the present invention made intensive studies to achieve the above objects and finally found that a composition (in particular, a particulate or granular resin composition) containing a resin (in particular, a thermoplastic resin) and an ultraviolet scattering agent surprisingly increases or improves in ultraviolet scattering function by deforming (e.g., crushing) the composition into a plate-like form. The present invention was accomplished based on the above findings.

That is, an aspect of the present invention provides a resin powder containing an ultraviolet scattering agent [a resin powder containing an ultraviolet scattering agent and a resin (a resin as a binder or a matrix)] and having being in a plate-like (platelet) form as defined in the claims .

The resin powder is a thermoplastic resin powder comprising a polyamide resin wherein the polyamide resin comprises an aliphatic homo- or co-polyamide having a C8 -16 alkylene chain.

In the resin powder, the ultraviolet scattering agent comprises metal oxide particles. The particulate ultraviolet scattering agent may have an average particle diameter (or average particle size) of not more than 150 nm. A representative ultraviolet scattering agent may include at least one metal oxide particle having an average particle diameter of not more than 100 nm and being selected from a titanium oxide particle and a zinc oxide particle.

In the resin powder, the ratio of the ultraviolet scattering agent is 50 to 150 parts by weight relative to 100 parts by weight of the polyamide resin forming the resin powder.

The resin powder has an average thickness (an average thickness determined from an electron micrograph) of, not more than 2 µm. The resin powder has a ratio of an average thickness and an average diameter (a ratio of an average thickness and an average diameter determined from an electron micrograph) in the former/the latter of about 1/5 to 1/200.

The resin powder is a powder (or a plate-like resin particle) obtainable by deforming (for example, crushing) a resin particle containing an ultraviolet scattering agent into a plate-like form. In the resin powder, the resin particle (the resin particle before deformation into a plate-like form) may have an average particle diameter of about 0.5 to 100 µm.

The resin powder may be a resin powder (a cosmetic resin powder) used for a cosmetic (or a cosmetic preparation).

Another aspect of the present invention provides a method for producing the resin powder (plate-like resin powder ), comprising: deforming a resin powder containing an ultraviolet scattering agent into a plate-like form.

Another aspect of the present invention also provides a cosmetic (or a cosmetic preparation) containing the resin powder (plate-like resin powder).

### EFFECTS OF THE INVENTION

The resin powder of the present invention has an ultraviolet scattering function. In particular, the resin powder of the present invention efficiently shows an ultraviolet scattering function although the ultraviolet scattering agent is contained or enclosed (or is not exposed) in the resin (or the ultraviolet scattering agent is not exposed) . Thus even a small quantity of the ultraviolet scattering agent suitably shows a sufficient ultraviolet scattering function.

Since the resin powder of the present invention contains the ultraviolet scattering agent enclosed in the resin, direct contact of the ultraviolet scattering agent with the skin is preventable or controllable. Further, as described above, the resin powder has an excellent the ultraviolet scattering function although the ultraviolet scattering agent is contained or dispersed in the resin. Thus the resin powder of the present invention has both high safety for the human body and excellent ultraviolet scattering function, and the resin powder is of much practical use and has a significant usefulness.

### BRIEF DESCRIPTION OF DRAWINGS

[Fig. 1] Fig. 1 is an electron micrograph of a plate-like powder (a plate-like resin powder containing a titanium oxide particle) obtained in Example 1.
[Fig. 2] Fig. 2 is an electron micrograph of a particle (powder) before deformation into a plate-like form in Example 1.
[Fig. 3] Fig. 3 is an electron micrograph of a plate-like powder (a plate-like resin powder containing a zinc oxide particle) obtained in Example 2.
[Fig. 4] Fig. 4 is an electron micrograph of a particle (powder) before deformation into a plate-like form in Example 2.

### DESCRIPTION OF EMBODIMENTS

### [Resin powder]

The resin powder of the present invention contains an ultraviolet scattering agent (an ultraviolet protective agent). In other words, the resin powder of the present invention contains an ultraviolet scattering agent and a resin. The resin powder is in a plate-like form as described below.

### (Resin)

The resin powder of the present invention contains a resin which is a polyamide resin wherein the polyamide resin comprises an aliphatic homo- or co-polyamide having a C8 -16 alkylene chain, Specifically, the resin powder of the present invention contains a resin as a matrix (or binder) and an ultraviolet scattering agent contained (or dispersed) in the matrix (or binder).

Also disclosed are a thermoplastic resin, a thermosetting resin (such as an epoxy resin, a silicone resin, or an unsaturated polyester resin) . In particular, the resin may at least comprise a thermoplastic resin.

Also disclosed is that the thermoplastic resin may include, for example, a polyamide resin {for example, an aliphatic polyamide [e.g., a polyamide 6, a polyamide 66, a polyamide 610, a polyamide 612, a polyamide 912, a polyamide 1212, a polyamide 1012, a polyamide 1010, a polyamide 11, a polyamide 12, and a copolyamide (e.g., a copolyamide in which polyamide components as exemplified above are copolymerized, such as a polyamide 66/11 or a polyamide 66/12)], an alicyclic polyamide, and an aromatic polyamide}, a polyester resin {for example, an aliphatic polyester resin (e.g. , a polylactic acid) and an aromatic polyester resin [e.g., a poly(alkylene arylate) (e.g., a poly(ethylene terephthalate), a poly(butylene terephthalate), a poly(ethylene naphthalate), and a poly(1,4-cyclohexanedimethylene terephthalate)), a polyarylate, and a liquid crystal polyester]}, a polycarbonate resin (for example, an aromatic polycarbonate, such as a bisphenol Abased polycarbonate), a poly(phenylene ether) resin (e.g., a poly(phenylene ether) and a modified poly(phenylene ether)), a polyetherketone resin (e.g., a polyetheretherketone, a polyetherketoneketone, a polyether-diphenyl-ether-phenyl-ketone-phenyl, and a polyetherketoneetherketoneketone), a polyacetal resin, a polysulfone resin (e.g., a polysulfone and a polyethersulfone), a polyimide resin (e.g., a polyimide, a polyamideimide, and a polyetherimide), a poly (phenylene sulfide) resin (e.g., a poly(phenylene sulfide)), an acrylic resin (e.g., a poly(methyl methacrylate)), a styrene-series resin (e.g., a polystyrene, and a styrene copolymer, such as an AS resin), an olefin resin [for example, a chain olefin resin (e.g., a polyethylene, a polypropylene, and a polymethylpentene), and a cyclic olefin resin (e.g., what is called a COP or a COC)], a vinyl-series resin (e.g., a vinyl ester-series resin, such as a poly (vinyl acetate), and a vinyl alcohol-series resin), a halogen-containing resin (e.g., a chlorine-containing resin, such as a poly (vinyl chloride) or a poly(vinylidene chloride), and a fluororesin), a cellulose-series resin (e.g., a cellulose acylate, such as a cellulose acetate), a thermoplastic elastomer [for example, an elastomer having a hard segment and a soft segment in a molecule thereof, e.g., a polyamide-series elastomer (e.g., a polyamide elastomer containing a polyamide component (e.g., an aliphatic polyamide component, such as a polyamide 6 or a polyamide 12) as a hard segment and a polyether component (e.g., a polyetherdiol) as a soft segment), a polyester-series elastomer, a polyurethane-series elastomer, a polyolefin-series elastomer, a polystyrene-series elastomer, and a fluorine-series elastomer].

These resins may be used alone or in combination.

The resin may be a water-soluble resin or may usually be a hydrophobic resin.

Among these resins, the thermoplastic resin is preferred. In particular, the polyamide resin may suitably be used. The polyamide resin is preferred in that the polyamide resin has a relatively high affinity with an ultraviolet scattering agent and that the polyamide powder seems to be efficiently and easily deformed into a plate-like particulate form by the after-mentioned deformation process. Among them, an aliphatic polyamide is widely used. In light of excellent formability or solvent resistance and easy deformation from a particulate form into a plate-like form, an aliphatic homo- or co-polyamide having a C₈₋₁₆alkylene chain (in particular, an aliphatic homo- or co-polyamide having a C₁₀₋₁₄alkylene chain, such as a polyamide 11 or a polyamide 12) is used in the present invention.

Even in a case where the resin powder of the present invention contains other additives or contacts with other additives (for example, in a case where the resin powder is used for cosmetic or other applications, as described below), the resin powder is relatively hardly attacked by other additives. Thus the resin powder is preferred in light of easy maintenance of an ultraviolet scattering function thereof.

The resin (a thermoplastic resin, in particular, a polyamide resin comprising an aliphatic homo- or co-polyamide having a C8 -16 alkylene chain,) may have a non-limiting number-average molecular weight to be selected according to the species of the resin or other factors. For example, the resin may have a number-average molecular weight of not less than 3000 (e.g., about 5000 to 1000000), preferably not less than 8000 (e.g., about 10000 to 500000), and more preferably not less than 15000 (e.g., about 20000 to 200000) in gel permeation chromatography (GPC) in terms of polystyrene.

In a case where the thermoplastic resin has a melting point (or softening point), the melting point (or softening point) is not particularly limited to a specific one. In particular, from the point of view of efficient deformation into a plate-like form in the presence of an ultraviolet scattering agent, the thermoplastic resin (in particular, a polyamide resin, such as an aliphatic polyamide resin) may have a melting point (or softening point) of not higher than 300°C (e.g., about 50 to 280°C) and preferably about 80 to 250°C (e.g., about 100 to 220°C) .

### (Ultraviolet scattering agent)

The ultraviolet scattering agent (ultraviolet protective agent) comprises metal oxide particles.

Concrete examples of the ultraviolet scattering agent may include an oxide [for example, a metal oxide, such as a metal oxide at least containing a group 4 metal of the Periodic Table as a metal component (e.g. , titanium oxide and zirconium oxide) or zinc oxide], a sulfide (e.g., a metal sulfide, such as zinc sulfide), a carbonate (e.g., calcium carbonate and barium carbonate), and a sulfate (e.g., barium sulfate). These ultraviolet scattering agents may be used alone or in combination. The ultraviolet scattering agent may have a function as a filler or a function as a coloring agent.

Among them, a metal oxide is preferred. A particularly preferred one includes titanium oxide and/or zinc oxide.

The titanium oxide may include, for example, titanium monoxide (TiO), titanium dioxide (TiO₂), and dititanium trioxide (Ti₂O₃). In particular, titanium dioxide is preferred. According to the present invention, different titanium oxides may be used in combination.

The titanium oxide may have any crystal form, such as a rutile form, an anatase form, or a brookite form. In particular, according to the present invention, rutile titanium oxide may preferably be used.

The ultraviolet scattering agent (for example, a metal oxide, such as titanium oxide) may be surface-treated with a surface-treating agent. The surface treatment allows the ultraviolet scattering agent (e.g., titanium oxide) to decrease in the reactivity (or activity) or to increase in the dispersibility to a resin and easily enables the ultraviolet scattering agent to function more efficiently.

The surface-treating agent may include a metal oxide (for example, silica and alumina) and an organic surface-treating agent [for example, a coupling agent (e.g., a silane coupling agent and a titanium coupling agent), an organic acid, an alcohol, and a siloxane-series compound]. These surface-treating agents may be used alone or in combination. In a case where the ultraviolet scattering agent contains a metal oxide, a metal oxide to be selected as the surface-treating agent is different from the metal oxide contained in the ultraviolet scattering agent. For example, in a case where the ultraviolet scattering agent contains titanium oxide, a non-titanium metal oxide is used as the surface-treating agent. In particular, the surface-treating agent may contain at least a metal oxide (e.g., silica).

For an ultraviolet scattering agent surface-treated (e.g., a titanium oxide surface-treated with a surface-treating agent), the proportion of the surface-treating agent in the ultraviolet scattering agent may be, for example, not more than 30% by weight (e.g., about 0.1 to 25% by weight), preferably not more than 20% by weight (e.g., about 0.5 to 18% by weight), and more preferably not more than 15% by weight (e.g., about 1 to 12% by weight) or may be about 1 to 20% by weight (e.g., about 2 to 15% by weight and preferably about 3 to 10% by weight).

The ultraviolet scattering agent is not particularly limited to a specific form. The ultraviolet scattering agent may have a particulate (or granular) form (including a spherical form), a fiber form (or a needle form or a rod form), or a plate form. A preferred form includes a particulate form.

The particulate ultraviolet scattering agent (for example, a metal oxide particle, such as a titanium oxide particle or a zinc oxide particle) may have an average particle diameter (or size) (an average primary particle diameter or an average dispersion particle diameter in a resin) selected from the range of, for example, about 1 to 1000 nm (e.g., about 2 to 800 nm) or may have an average particle diameter of about 3 to 500 nm, preferably about 5 to 400 nm (e.g., about 7 to 350 nm), and more preferably about 10 to 300 nm (e.g., about 15 to 250 nm). In particular, the particulate ultraviolet scattering agent may have an average particle diameter of not more than 200 nm (e.g., about 1 to 180 nm), preferably not more than 150 nm (e.g., about 5 to 120 nm), more preferably not more than 100 nm (e.g., about 10 to 80 nm), and particularly not more than 50 nm (e.g., about 20 to 50 nm). According to the present invention, even the ultraviolet scattering agent having such a small particle diameter is safe for the human body. Thus since the ultraviolet scattering agent has both relatively high transparency and high ultraviolet scattering characteristic and is safe for the human body, the ultraviolet scattering agent is preferred.

The average particle diameter of the ultraviolet scattering agent can be measured by a conventional method. The average particle diameter may be measured by the same method as a method for measuring the average diameter of the resin powder described later.

The ultraviolet scattering agent may be compatible with the resin. The ultraviolet scattering agent may usually be incompatible with the resin.

The ratio of the ultraviolet scattering agent (in particular, a metal oxide, such as titanium oxide) relative to 100 parts by weight of the resin is 50 to 150 parts by weight.

The volume proportion of the ultraviolet scattering agent (in particular, a metal oxide, such as titanium oxide) in the total volume of the resin and the ultraviolet scattering agent may be, for example, about 0.1 to 50% by volume (e.g. , about 0.5 to 45% by volume), preferably about 1 to 40% by volume (e.g., about 1.5 to 35% by volume), more preferably about 2 to 30% by volume (e.g., about 3 to 25% by volume), and usually about 3 to 50% by volume (e.g., about 5 to 45% by volume, preferably about 8 to 40% by volume, and more preferably about 10 to 35% by volume).

### (Other additives)

The resin powder of the present invention may contain other additives (additives other than the ultraviolet scattering agent) if necessary. Other additives can suitably be selected as usage and, for example, may include a stabilizer (e.g., an ultraviolet absorber), a filler, a coloring agent, a dispersing agent, an emulsifier, a perfume, a preservative, an antioxidant, a medicinal component, an extender, a defoaming agent, a humectant, and a lubricant. These additives may be used alone or in combination.

The ultraviolet absorber may include, for example, an organic compound, such as an aminobenzoic acid (e.g., p-aminobenzoic acid) or an ester thereof, an ester of salicylic acid, an ester of cinnamic acid (e.g., benzyl cinnamate and 2-ethylhexyl methoxycinnamate), an ester of a dialkylaminohydroxybenzoylbenzoic acid (e.g., hexyl diethylaminohydroxybenzoylbenzoate), a benzophenone-series compound (e.g., 2-hydroxy-4-methoxybenzophenone), a dibenzoylalkane (e.g., 4-tert-butyl-4-methoxydibenzoylmethane), a triazine derivative (e.g., 2,4-bis{[4-(2-ethylhexyloxy)-2-hydroxy]-phenyl}-6-(4-methoxyphenyl)-1,3,5-triazine), urocanic acid or an ester thereof, or β-carotene. These ultraviolet absorbers may be used alone or in combination.

The filler may be an inorganic filler or an organic filler. The filler may have any form, such as a particulate form, a fiber form, or a needle form. Concrete examples of the filler may include a carbonate (such as calcium carbonate or magnesium carbonate), a sulfate (such as calcium sulfate or barium sulfate), a phosphate (such as calcium phosphate or titanium phosphate), a metal oxide (such as silica, alumina, iron oxide, magnesium oxide, strontium oxide, or cerium oxide), a hydroxyapatite, a silicate (such as mica, calcium silicate, wollastonite, bentonite, zeolite, maifan stone, talc, montmorillonite, clay, or kaolin), a mineral (such as quartz powder, diatomaceous earth, nepheline syenite, cristobalite, or dolomite), a metal nitride (such as silicon nitride, boron nitride, aluminum nitride, or titanium nitride), a metal hydroxide (such as aluminum hydroxide), a metal carbide (such as silicon carbide, boron carbide, titanium carbide, or tungsten carbide), a metal boride (such as titanium boride or zirconium boride), a metal [for example, gold, platinum, palladium, and a ferromagnetic metal or alloy (e.g., iron, cobalt, nickel, magnetite, and ferrite)], a carbon (such as carbon black, graphite, or carbon nanotube), a glass (such as glass powder, glass bulb, hollow glass bulb, or glass flake), a crosslinked resin (e.g., a crosslinked poly(methyl methacrylate)), a fiber (or fibrous) filler (e.g., a carbon fiber), and a filler to which a metal having an antibacterial function (such as silver, copper, or zinc) is supported (e.g., hydroxyapatite silver and zeolite silver). These fillers may be used alone or in combination.

The coloring agent may be inorganic or organic. The coloring agent may also be a dye or a pigment (or a dye and pigment) . Concrete examples of the coloring agent may include an inorganic coloring agent (or an inorganic pigment, e.g., carbon black, ultramarine blue, Indian red (red iron oxide), black iron oxide, yellow iron oxide, chromium oxide, and a complex oxide pigment), and an organic coloring agent (e.g., an azo-series pigment, a phthalocyanine-series pigment (such as copper phthalocyanine blue or copper phthalocyanine green), an isoindolinone-series pigment, a perynone-perylene-series pigment, a thren-series pigment, a dioxazine pigment, an anthraquinone-series pigment, an indigo-series pigment, a thioindigo-series pigment, a diketopyrrolopyrrole-series pigment, a benzimidazolone-series pigment, a quinacridone-series pigment, an oil-based dye, and a disperse dye). The coloring agent may also include a tar dye (e.g., a tar dye for cosmetic preparation prescribed by Ordinance of the Ministry of Health, Labor and Welfare), and a lake pigment (such as an aluminum lake) . These coloring agents may be used alone or in combination.

### (Form of resin powder)

The resin powder of the present invention has a plate-like (flat-plate or platelet) form. The plate-like resin powder (plate-like primary particle) has an average thickness of not more than 2 µm (e.g. , about 0.1 to 1.8 µm), more preferably not more than 1.5 µm (e.g. , about 0.15 to 1.3 µm), and particularly not more than 1.2 µm (e.g., about 0.2 to 1.2 µm).

The average thickness is determined from an electron micrograph.

Specifically, the thickness (maximum thickness) of each of a plurality of [for example, not less than 10 (e.g. , 15 to 100, preferably 20 to 50)] resin powders randomly extracted (sampled) from an electron micrograph is measured, and the average of the resulting thickness values can be expressed as the average thickness. The average diameter of the resin powder [the average of a length in the plane direction (or the direction perpendicular to the thickness direction) of the plate-like primary particle] can be selected from the range of about 2 to 700 µm (e.g., about 5 to 600 µm). For example, the average diameter may be about 10 to 500 µm, preferably about 15 to 200 µm, and more preferably about 20 to 150 µm.

The average diameter is determined from an electron micrograph in the same manner as the average thickness. Specifically, the diameter or length (maximum diameter or maximum length or major axis) of each of a plurality of resin powders randomly extracted (sampled) from an electron micrograph is measured, and the average of the resulting diameter or length values can be expressed as the average diameter (average length).

For the plate-like resin powder, the ratio of the average thickness relative to the average diameter (the former/the latter) is from 1/5 to 1/200, and more preferably about 1/10 to 1/150 (e.g., 1/15 to 1/100) or may usually be about 1/5 to 1/100 (e.g., about 1/8 to 1/80, preferably about 1/10 to 1/60, and more preferably about 1/15 to 1/50) . The average thickness and the average diameter to be used in the ratio may be the values measured as described above.

It is also disclosed that the diameter of the resin powder or the diameter distribution thereof can be determined (measured) by a light-scattering (dynamic light-scattering) method. For example, the diameter (the diameter distribution) of the resin powder determined by a light-scattering method may be, for example, about 0.01 to 700 µm (e.g., about 0.05 to 600 µm), preferably about 0.1 to 500 µm (e.g., about 0.2 to 400 µm), and more preferably 0.3 to 300 µm (e.g., about 0.5 to 200 µm).

The proportion of the resin powder having a diameter showing the maximum frequency determined by a light-scattering method may be, for example, about 3 to 30%, preferably about 5 to 25%, and more preferably about 7 to 20% (e.g., about 10 to 15%).

In particular, as described later, the resin powder of the present invention may be a powder (an aggregate of plate-like particles) obtainable by deforming a plurality of resin particles (an aggregate of resin particles, or an agglomerated resin particle; hereinafter may simply be referred to as a resin particle) containing (or enclosing) an ultraviolet scattering agent (and optionally other additives described above) into a plate-like form.

For the powder, usually, the resin particle (each resin particle, single resin particle) may independently be deformed into a plate-like form (or may have a plate-like form). According to the resin powder of the present invention, a plurality of plate-like resin particles may be aggregated or agglomerated (or laminated or stacked). The resin particles usually have a weak cohesive force and are practically separated easily in synthesis or in use.

### (Method for producing resin powder)

The method for producing the resin powder (plate-like resin powder) of the present invention comprises (A) a method of deforming a resin powder containing an ultraviolet scattering agent (a resin powder that has not been deformed into a plate-like form; hereinafter may simply be referred to as a resin powder) into a plate-like form (or a plate-like powder). Also disclosed is (B) a method of pulverizing (or crushing) a film (or a sheet or a film-like product) composed of a resin containing an ultraviolet scattering agent. According to the present invention, the method (A) is used. In the method (A), it is not necessary to form a film, and a powder having relatively less variation in size (thickness or particle diameter) is efficiently obtainable.

For the method (A), the resin powder containing an ultraviolet scattering agent may be obtainable by pulverizing (e.g., freeze-pulverizing) a resin composition (a pellet resin composition) containing an ultraviolet scattering agent. In particular, a resin particle containing an ultraviolet scattering agent may preferably be used.

The resin particle to be used may be a commercially available product or may be produced by using or applying a known method. The known method may include, for example, a method (forced emulsification) of kneading a resin as a matrix, an ultraviolet scattering agent (and other additives), and a third component (for example, a sugar and a poly (ethylene glycol)) incompatible with the matrix (and the ultraviolet scattering agent) and easily removable by a medium, such as water, to give a composition composed of the third component and the resin particle containing the ultraviolet scattering agent, and removing the third component from the composition (for example, a method described in Japanese Patent Application Laid-Open Publication No. 2010-132811).

The form of the resin particle may include, but should not be limited to, a spherical form, an ellipsoidal form, and others. In particular, the form of the resin particle may be a substantially spherical form (particularly, a spherical form).

The resin particle (the resin particle before deformation into a plate-like form) may have an average particle diameter selected from the range of about 0.1 to 500 µm, and, for example, may have an average particle diameter of about 0.2 to 300 µm (e.g., about 0.3 to 200 µm), preferably about 0.5 to 100 µm (e.g., about 1 to 70 µm), more preferably about 2 to 50 µm (e.g., about 3 to 40 µm), and usually about 4 to 30 µm. In particular, the resin particle may have an average particle diameter of not more than 50 µm, preferably not more than 30 µm, more preferably not more than 20 µm, and particularly preferably not more than 15 µm. The resin particle having such a particle diameter is easy to deform into a plate-like form efficiently.

For the method (A), the method of deforming the resin powder into a plate-like form may include, but should not be limited to, crushing the resin powder (in particular, the resin particle) . According to the present invention, a plate-like product is obtainable surprisingly without cracks by crushing the resin powder (in particular, a resin particle, such as an aliphatic polyamide resin) . For the method, various apparatuses or means that can crush the resin powder by a physical force (an apparatus or means for deformation into a plate-like form) is utilizable. The apparatus or means may include a mill (a media dispersing machine), a roll [such as a mill roll (such as a two-roll mill or a three-roll mill)], and a media-less dispersing machine [for example, a high-pressure collision dispersing machine (such as a nanomizer or an artimizer) and an ultrasonic dispersing machine].

Among them, the mill is user-friendly due to relatively excellent operationality.

The mill (media mill) is classified broadly into two groups: what is called a wet media mill and a dry media mill; the wet media mill uses a medium including a liquid, and the dry media mill does not use a liquid. According to the present invention, both media mills are available.

The wet media mill may include a ball mill, a side grinder, a dyno mill, a spike mill, a DCP mill, a basket mill, and a paint conditioner. The dry media mill may include a ball mill, a vibrating ball mill, an attritor, and a dry bead mill.

The material of a container to be used for the media mill may include, but should not be limited to, a hardened steel, a stainless steel, a SUS chrome plating, an alumina ceramic, a silicon nitride ceramic, a zirconia ceramic, a silicon carbide ceramic, a zirconia-toughened alumina ceramic, and a Sialon.

As a media particle to be used for the media mill, there may usually be employed a particle having a spherical form (shape). The material of the media particle may include a glass bead, a low-alkali glass bead, a no-alkali glass bead, an alumina bead, a zirconia bead, a zirconia yttria bead, a titania bead, a high-purity alumina bead, and a steel ball. The media particle (bead) may have a specific gravity of, for example, not less than 2.0, preferably not less than 2.5, and more preferably not less than 3.0.

The size of the media particle (bead) can suitably be selected according to the size of the resin particle to be subjected to deformation into a plate-like form, or other factors. The media particle (bead) may have a size of, for example, about 0.05 to 5 mm (e.g., about 0.1 to 3 mm).

For the wet media mill, water may usually be employed as the media (liquid) . The media (liquid) to be used may also include a mixed solvent containing water and an aqueous solvent [or a water-soluble solvent, for example, an alcohol (an alkanol, such as methanol, ethanol, or isopropyl alcohol; a diol, such as 1,2 -pentanediol or 1,2-hexanediol), a diol monoether (e.g., an alkylene glycol monoalkyl ether, such as ethylene glycol monomethyl ether or propylene glycol monobutyl ether; and a polyalkylene glycol monoalkyl ether, such as diethylene glycol monoethyl ether or triethylene glycol monobutyl ether)].

### [Use of resin powder]

The resin powder (plate-like resin powder) of the present invention can be used for various purposes that in need of an ultraviolet scattering function. In particular, the resin powder can preferably be used for a cosmetic (or a cosmetic preparation).

Thus, the present invention also includes a cosmetic (or a cosmetic preparation) containing the resin powder. The cosmetic (in particular, a skin cosmetic) contains at least the resin powder and may further a known cosmetic component (for example, an oil, an alcohol, a thickener, and other additives as described above).

Other additives may be contained in the resin powder as described above or may be contained in the cosmetic independently of the resin powder.

The cosmetic may have any form, such as a liquid form (such as a lotion, an emulsion, a cream, or a gel), a semi-solid form (such as a cream, a gel, or a paste), or a solid form (such as a particle, a powder, a molded product having a desired form).

### EXAMPLES

The following examples are intended to describe this invention in further detail and should by no means be interpreted as defining the scope of the invention. Measurement methods of physical properties are as follows.

### (Average thickness and average diameter of resin powder)

A sample (9.5 g) was washed with 2L distilled water three times, filtered, and dried by a vacuum dryer.

The powder adhering on the filter was sampled (collected) at random three points on the filter by a spatula and placed on an observation table. Gold was deposited onto the sample placed on the observation table .

The resulting sample was measured and observed in a 3D mode at prescribed magnifications (1000, 2000, 3000, and 5000 magnifications) by a scanning electron microscope (SEM) VE-8800 manufactured by KEYENCE Corporation.

From the resulting electron micrograph, 60 particles in the observation field were selected (30 particles which were easy to measure in the thickness direction, and 30 particles which were easy to measure in the longitudinal direction (plate surface direction)). The thickness (the maximum thickness) or the diameter (the maximum length in the longitudinal direction) of each particle was measured by an analysis mode. For particles before deformation into a plate-like form, only 30 particles were selected, and the particle diameter of each particle was measured.

In some cases, the plate-like particles were apparently aggregated under the above-mentioned measurement conditions, and the thickness or diameter of each particle was hard to measure. Thus the magnification was suitably selected to facilitate the measurement.

### (Diameter (or diameter distribution) of resin powder)

A sample was dispersed in water, and the diameter (diameter distribution) of the sample was measured by a laser diffraction/scattering particle diameter distribution measuring apparatus (LA920, manufactured by Horiba, Ltd.).

### (Example 1)

Fifty (50) parts by weight of a titanium oxide particle [titanium dioxide, manufactured by Dupont, Ti-Pure(R) Titanium Dioxide Pigment - Paint Coatings-DryGrades R-105, surface-treated (silica, aluminadimethylsiloxane-treated) product] and 50 parts by weight of a polyamide resin (polyamide 12, manufactured by Daicel-Evonik Ltd.) were provided. In the same manner as Example 1 of Japanese Patent Application Laid-Open Publication No. 2005-179646, a polyamide particle containing the titanium oxide particle was obtained (spherical form, average particle diameter: 4.1 µm, proportion of titanium oxide particle: 50% by weight).

The resulting particle was dispersed in water in a proportion of 10% by weight, and in this state the particle was crushed by a ball mill to give a plate-like powder. As the media species of the ball mill for this process, a high-purity alumina bead (particle diameter: 0.5 mm) was used. The volume of the dispersion and that of the media were the same.

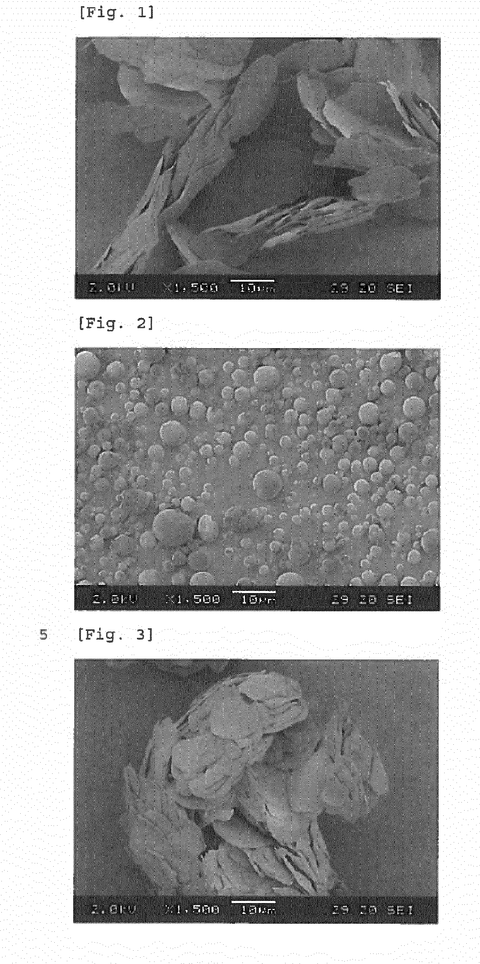

The electron micrograph (1500 magnifications) of the resulting plate-like powder is shown in Fig. 1. For comparison, the electron micrograph (1500 magnifications) of the unprocessed polyamide particle containing the titanium oxide particle (the polyamide particle before deformation into a plate-like form) is shown in Fig. 2. Apparent from these photographs, each polyamide particle shown in Fig. 1 was in the form of a plate.

The plate-like powder had an average thickness of 0.94 µm, an average diameter of 32 µm, a diameter distribution of 7 to 110 µm, a diameter showing the maximum frequency of 26 µm, and a proportion of a diameter showing the maximum frequency of 12%.

The resulting plate-like powder was dispersed in ethanol in a proportion of 5% by weight, and the resulting dispersion was applied on a slide glass by a bar coater.

The ethanol was dried naturally, and then the ultraviolet (UV) transmittance was measured by an UV-measuring apparatus. The UV transmittance measured 40% at 300 nm, 38% at 330 nm, 38% at 360 nm, and 37% at 400 nm.

The UV transmittance of the unprocessed polyamide particle containing the titanium oxide particle (polyamide particle before deformation into a plate-like form) was measured in the same manner. The UV transmittance measured 66% at 300 nm, 66% at 330 nm, 68% at 360 nm, and 65% at 400 nm.

Thus it was found that the deformation into a plate-like form significantly reduced the UV transmittance of the polyamide particle and greatly improved the ultraviolet-preventing function of the polyamide particle.

### (Example 2)

A polyamide particle containing a zinc oxide particle was obtained (spherical form, average particle diameter: 4.5 µm, proportion of zinc oxide particle: 50% by weight) in the same manner as Example 1 except that 50 parts by weight of a zinc oxide particle (average particle diameter: 20 nm, manufactured by Sakai Chemical Industry Co., Ltd., "FINEX-50S-LP2", surface-treated (organopolysiloxane) product) was used instead of the titanium oxide in Example 1.

The resulting particle was deformed in the same manner as Example 1 to give a plate-like powder.

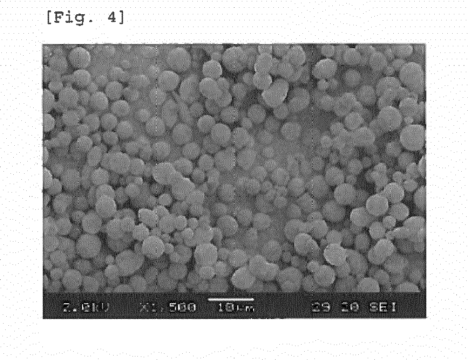

The electron micrograph (1500 magnifications) of the resulting plate-like powder is shown in Fig. 3. For comparison, the electron micrograph (1500 magnifications) of the unprocessed polyamide particle containing the zinc oxide particle (the polyamide particle before deformation into a plate-like form) is shown in Fig. 4. Apparent from these photographs, each polyamide particle shown in Fig. 3 was in the form of a plate.

The plate-like powder had an average thickness of 1.00 µm, an average diameter of 25 µm, a diameter distribution of 5 to 215 µm, a diameter showing the maximum frequency of 12 µm, and a proportion of a diameter showing the maximum frequency of 13%.

In the same manner as Example 1, the UV transmittance measured 0% at 300 nm, 4% at 330 nm, 6% at 360 nm, and 15% at 400 nm.

The UV transmittance of the unprocessed polyamide particle containing the zinc oxide particle (polyamide particle before deformation into a plate-like form) was measured in the same manner. The UV transmittance measured 10% at 300 nm, 20% at 330 nm, 22% at 360 nm, and 27% and 400 nm.

Thus it was also found that the deformation into a plate-like form significantly reduced the UV transmittance of the polyamide particle containing the zinc oxide and greatly improved the ultraviolet-preventing function of the polyamide particle.

### (Example 3)

A polyamide particle containing a titanium oxide particle was obtained (spherical form, average particle diameter: 4.5 µm, proportion of titanium oxide particle: 55% by weight) in the same manner as Example 1 except that the proportion of the polyamide resin was changed from 50 parts by weight to 40 parts by weight in Example 1.

The resulting particle was deformed in the same manner as Example 1 to give a plate-like powder. The plate-like powder had an average thickness of 1.1 µm and an average diameter of 29 µm.

In the same manner as Example 1, the UV transmittance measured 28% at 300 nm, 28% at 330 nm, 28% at 360 nm, and 29% at 400 nm.

The UV transmittance of the unprocessed polyamide particle containing the titanium oxide particle (polyamide particle before deformation into a plate-like form) was measured in the same manner. The UV transmittance measured 42% at 300 nm, 42% at 330 nm, 40% at 360 nm, and 41% at 400 nm.

### (Example 4)

A polyamide particle containing a titanium oxide particle was obtained (spherical form, average particle diameter: 5.7 µm, proportion of titanium oxide particle: 60% by weight) in the same manner as Example 1 except that the proportion of the titanium oxide particle was changed from 50 parts by weight to 60 parts by weight and that the proportion of the polyamide resin was changed from 50 parts by weight to 40 parts by weight in Example 1.

The resulting particle was deformed in the same manner as Example 1 to give a plate-like powder. The plate-like powder had an average thickness of 1.1 µm and an average diameter of 24 µm.

In the same manner as Example 1, the UV transmittance measured 28% at 300 nm, 27% at 330 nm, 28% at 360 nm, and 26% at 400 nm.

The UV transmittance of the unprocessed polyamide particle containing the titanium oxide particle (polyamide particle before deformation into a plate-like form) was measured in the same manner. The UV transmittance measured 42% at 300 nm, 40% at 330 nm, 39% at 360 nm, and 38% at 400 nm.

### (Example 5)

A polyamide particle containing a zinc oxide particle was obtained (spherical form, average particle diameter: 4.7 µm, proportion of zinc oxide particle: 55% by weight) in the same manner as Example 2 except that the proportion of the polyamide resin was changed from 50 parts by weight to 40 parts by weight in Example 2.

The resulting particle was deformed in the same manner as Example 1 to give a plate-like powder. The plate-like powder had an average thickness of 1.0 µm and an average diameter of 33 µm.

In the same manner as Example 1, the UV transmittance measured 0% at 300 nm, 4% at 330 nm, 6% at 360 nm, and 11% at 400 nm.

The UV transmittance of the unprocessed polyamide particle containing the zinc oxide particle (polyamide particle before deformation into a plate-like form) was measured in the same manner. The UV transmittance measured 0% at 300 nm, 9% at 330 nm, 15% at 360 nm, and 15% at 400 nm.

### (Example 6)

A polyamide particle containing a zinc oxide particle was obtained (spherical form, average particle diameter: 6.7 µm, proportion of zinc oxide particle: 60% by weight) in the same manner as Example 2 except that the proportion of the zinc oxide particle was changed from 50 parts by weight to 60 parts by weight and that the proportion of the polyamide resin was changed from 50 parts by weight to 40 parts by weight in Example 2.

The resulting particle was deformed in the same manner as Example 1 to give a plate-like powder. The plate-like powder had an average thickness of 1.0 µm and an average diameter of 31 µm.

In the same manner as Example 1, the UV transmittance measured 0% at 300 nm, 2% at 330 nm, 4% at 360 nm, and 10% at 400 nm.

The UV transmittance of the unprocessed polyamide particle containing the zinc oxide particle (polyamide particle before deformation into a plate-like form) was measured in the same manner. The UV transmittance measured 0% at 300 nm, 8% at 330 nm, 14% at 360 nm, and 16% at 400 nm.

### INDUSTRIAL APPLICABILITY

The resin powder of the present invention has an ultraviolet scattering function and is usable for various purposes that require the ultraviolet scattering function. In particular, the resin powder is suitably useful for a cosmetic (particularly, skin cosmetic) application.

The cosmetic (or cosmetic preparation) containing the resin powder of the present invention includes, but should not be limited to, a cream (such as a facial cream, a body-care cream, or a lip balm), a foundation, a powder (such as a face powder), an eye shadow, an eye liner, a mascara, a sunburn preventive, a skin lotion, a milky lotion (an emulsion), an essence, a facial scrub, and a facial pack.

## Claims

1. A polyamide resin powder comprising a polyamide resin and an ultraviolet scattering agent, the polyamide resin powder being in a plate-like form and having an average thickness of not more than 2 µm determined from an electron micrograph and a ratio of an average thickness and an average diameter in the former/the latter of 1/5 to 1/200 determined from an electron micrograph,
wherein the polyamide resin comprises an aliphatic homo- or co-polyamide having a C₈₋₁₆ alkylene chain,
wherein the ultraviolet scattering agent comprises metal oxide particles, and
wherein the ratio of the ultraviolet scattering agent is 50 to 150 parts by weight relative to 100 parts by weight of the polyamide resin.

2. The polyamide resin powder according to claim 1, wherein the ultraviolet scattering agent comprises particles having an average particle diameter of not more than 150 nm.

3. The polyamide resin powder according to claim 1 or 2, wherein the ultraviolet scattering agent comprises metal oxide particles having an average particle diameter of not more than 100 nm, and the metal oxide particles comprise at least one member selected from the group consisting of titanium oxide particles and zinc oxide particles.

4. The polyamide resin powder according to any one of claims 1 to 3, which is obtainable by deforming resin particles, each comprising an ultraviolet scattering agent, into plate-like forms.

5. The polyamide resin powder according to claim 4, wherein the resin particles have an average particle diameter of 0.5 to 100 µm.

6. The polyamide resin powder according to any one of claims 1 to 5, which is used for a cosmetic.

7. A method for producing the polyamide resin powder according to any one of claims 1 to 6, comprising: deforming a polyamide resin powder comprising an ultraviolet scattering agent into a plate-like form.

8. A cosmetic comprising the polyamide resin powder according to any one of claims 1 to 6.

## Patentansprüche

1. Polyamidharzpulver, umfassend ein Polyamidharz und ein Ultraviolettstrahlung streuendes Mittel, wobei das Polyamidharzpuler in einer plättchenartigen Form vorliegt und eine mittlere Dicke von nicht mehr als 2 µm, bestimmt aus einer elektronenmikroskopischen Aufnahme, und ein Verhältnis von einer mittleren Dicke und einem mittleren Durchmesser in ersteres/letzterem von 1/5 bis 1/200, bestimmt aus einer elektronenmikroskopischen Aufnahme, aufweist,
wobei das Polyamidharz ein aliphatisches Homo- oder Co-Polyamid mit einer C₈₋₁₆-Alkylenkette umfasst,
wobei das Ultraviolettstrahlung streuende Mittel Metalloxidpartikel umfasst und
wobei das Verhältnis des Ultraviolettstrahlung streuenden Mittels 50 bis 150 Gewichtsanteile relativ zu 100 Gewichtsanteilen des Polyamidharzes beträgt.

2. Polyamidharzpulver gemäß Anspruch 1, wobei das Ultraviolettstrahlung streuende Mittel Partikel umfasst, die einen mittleren Partikeldurchmesser von nicht mehr als 150 nm aufweisen.

3. Polyamidharzpulver gemäß Anspruch 1 oder 2, wobei das Ultraviolettstrahlung streuende Mittel Metalloxidpartikel umfasst, die einen mittleren Partikeldurchmesser von nicht mehr als 100 nm aufweisen, und die Metalloxidpartikel mindestens ein Mitglied umfassen, ausgewählt aus der Gruppe, bestehend aus Titanoxidpartikeln und Zinkoxidpartikeln.

4. Polyamidharzpulver gemäß einem der Ansprüche 1 bis 3, das zu erhalten ist durch Verformen von Harzpartikeln, die jeweils ein Ultraviolettstrahlung streuendes Mittel umfassen, in plättchenartige Formen.

5. Polyamidharzpulver gemäß Anspruch 4, wobei die Harzpartikel einen mittleren Partikeldurchmesser von 0,5 bis 100 µm aufweisen.

6. Polyamidharzpulver gemäß einem der Ansprüche 1 bis 5, das für ein Kosmetikum verwendet wird.

7. Verfahren zum Herstellen des Polyamidharzpulvers gemäß einem der Ansprüche 1 bis 6, umfassend: Verformen eines Polyamidharzpulvers, umfassend ein Ultraviolettstrahlung streuendes Mittel, in eine plättchenartigen Form.

8. Kosmetikum, umfassend das Polyamidharzpulver gemäß einem der Ansprüche 1 bis 6.

## Revendications

1. Poudre de résine de polyamide comprenant une résine de polyamide et un agent de diffusion du rayonnement ultraviolet, la poudre de résine de polyamide se présentant sous une forme de plaque et ayant une épaisseur moyenne de pas plus de 2 µm déterminée à partir d'un micrographe électronique et un rapport d'une épaisseur moyenne et d'un diamètre moyen dans la précédente/la dernière de 1/5 à 1/200 déterminé à partir d'un micrographe électronique,
où la résine de polyamide comprend un homo- ou copolyamide aliphatique ayant une chaîne alcylène en C₈ à C₁₆,
où l'agent de diffusion du rayonnement ultraviolet comprend des particules d'oxyde métallique, et
où le rapport de l'agent de diffusion du rayonnement ultraviolet est de 50 à 150 parties en poids par rapport à 100 parties en poids de la résine de polyamide.

2. Poudre de résine de polyamide selon la revendication 1, dans laquelle l'agent de diffusion du rayonnement ultraviolet comprend des particules ayant un diamètre moyen de particule de pas plus de 150 nm.

3. Poudre de résine de polyamide selon la revendication 1 ou 2, dans laquelle l'agent de diffusion du rayonnement ultraviolet comprend des particules d'oxyde métallique ayant un diamètre moyen de particule de pas plus de 100 nm, et les particules d'oxyde métallique comprennent au moins un élément sélectionné dans le groupe constitué des particules d'oxyde de titane et des particules d'oxyde de zinc.

4. Poudre de résine de polyamide selon l'une quelconque des revendications 1 à 3, qui peut être obtenue en déformant les particules de résine, comprenant chacune un agent de diffusion du rayonnement ultraviolet, sous des formes type plaque.

5. Poudre de résine de polyamide selon la revendication 4, dans laquelle les particules de résine présentent un diamètre moyen de particule de 0,5 à 100 µm.

6. Poudre de résine de polyamide selon l'une quelconque des revendications 1 à 5, qui est utilisée pour un produit cosmétique.

7. Procédé de production de la poudre de résine de polyamide selon l'une quelconque des revendications 1 à 6, comprenant : la déformation d'une poudre de résine de polyamide comprenant un agent de diffusion du rayonnement ultraviolet en une forme type plaque.

8. Produit cosmétique comprenant la poudre de résine de polyamide selon l'une quelconque des revendications 1 à 6.
